(19) Europäisches Patentamt
European Patent Office
Office européen des brevets

(11) Publication number: **0 085 167**
**A1**

# (12) EUROPEAN PATENT APPLICATION

(21) Application number: 82111641.5

(22) Date of filing: 15.12.82

(51) Int. Cl.³: C 07 C 41/03
B 01 J 31/04

(30) Priority: 24.12.81 US 334108

(43) Date of publication of application:
10.08.83 Bulletin 83/32

(84) Designated Contracting States:
BE DE FR GB SE

(71) Applicant: CONOCO INC.
1000 South Pine Street P.O. Box 1267
Ponca City Oklahoma 74601(US)

(72) Inventor: Yang, Kang
2501 Robin Road
Ponca City, OK 74601(US)

(72) Inventor: Nield, Gerald Lee
837 Edgewood
Ponca City, OK 74601(US)

(72) Inventor: Washecheck, Paul Howard
2801 Canterbury
Ponca City, OK 74601(US)

(74) Representative: Patentanwälte Dipl.-Ing. A. Grünecker, Dr.-Ing. H. Kinkeldey, Dr.-Ing. W. Stockmair, Dr. rer. nat. K. Schumann, Dipl.-Ing. P.H. Jakob, Dr. rer. nat. G. Bezold Maximilianstrasse 58
D-8000 München 22(DE)

## (54) Alkoxylation with calcium and magnesium salts.

(57) Magnesium and calcium carboxylates are used for the alkoxylation of alcohols and alkyl phenols. The resulting alkoxylates have less free unreacted materials, sharper alkoxide homolog distributions and lower pour points as compared to commercial alkali base catalyzed alkoxylates.

EP 0 085 167 A1

Croydon Printing Company Ltd

# ALKOXYLATIONS WITH CALCIUM AND MAGNESIUM SALTS

This invention relates to the production of alkoxylated alkyl phenols and alkoxylated alcohols by reacting said alkyl phenols or alcohols in the presence of calcium and magnesium carboxylate catalysts. More specifically, this invention relates to the production of these alkoxylated materials by reacting them in the presence of these calcium carboxylates and/or magnesium carboxylates with adducting materials such as ethylene oxide and propylene oxide.

The general reaction of alcohols and ethylene oxide or propylene oxide to form alkoxylated alcohols, most commonly ethylene oxide adducts, has long been known and practiced on a commercial scale. For example, these ethylene oxide adducts have been used as detergents and cleaning agents, domestic and industrial laundry detergents, detergent builders, polishes, sanitizers and dry-cleaning materials. Other uses include the pulp and paper industry and the fiber industry. These materials are especially adapted to these uses since they have functional properties such as wetting power, foaming, emulsifying and dispersing abilities as well as solubilization and detergent abilities to facilitate their use.

Alkoxylated alkyl phenols are especially useful in the pulp and paper industry. During cellulose production, the alkyl phenol adducts are especially effective in the reduction of resin content where such materials accelerate the drainage of liquor from digesters and aid in the removal of resin from pulp. Belgium Patent 623,458 discloses the use of alkyl phenol adducts as a component in an especially desirable newspaper printing ink. In addition, these materials are useful in the textile industry as a detergent component for reducing a residual fat content of wool, scouring of wool and in sizing compounds and the like. These materials are also used for countering dry rot and potato tubers according to British Patent 773,798. In addition, pharmaceutical and

cosmetic preparations make use of the special properties of alkyl phenol adducts. These materials are also useful in the formulation of engine oil additives and specialty industrial lubricants.

The ethoxylation of alcohols is well known. French Patent 1,365,945 teaches the use of compounds containing an active hydrogen atom reacted with ethylene oxide in the presence of alkali metal base. Acidic catalysts in general are also known. However, the ethoxylation of alcohols inevitably produces a distribution of various adducts. For example, in surfactant applications an adduct with too few ethylene oxide molecules is not effective because of poor solubility, while an adduct with too many ethylene oxide molecules is likewise undesirable because surface tension reduction per unit mass decreases drastically with increasing molecular weight. Thus it has long been essential to produce and use adducts with a sharp distribution in the desired mole adduct range (5 to 10) as possible. Acid catalyzed reactions that are commercially used produce such ethoxylates but also produce harmful side products such as dioxane which must be removed prior to use. In addition, the distribution of these adducts is undesirably broad, leading to many adductive molecules, both above and below the desired adduct range.

The same problem with broad distribution and by-products is true in the adducting of alkoxylating materials onto alkyl phenols.

Russian Patent 523,074 teaches that alkali metals and various carbonates can be used to catalyze adducting reactions. The side product formation in the based catalyzed reactions is very low, but in most base catalyzed reactions the adduct distribution is undesirably broad. The result is that a large proportion of the product obtained is not useful or is less desirable because of distribution. Metal carbonates normally used are sodium and potassium carbonates.

Representative of but non-exhaustive of art in this area is U.S. Patent 3,328,467 which describes the use of zeolites and modified zeolites as catalysts in the ethoxylation reactions. French Patent 1,557,407 teaches triethyl oxonium fluoroborate to catalyze such reactions. Many art references exist teaching alkali metal hydroxides such as sodium and potassium hydroxide, tertiary amines and sodium metal.

U.S. Patent 4,239,917 teaches the use of barium oxide catalyzed ethoxylation of alcohols to produce a narrow distribution without undue by-products and utilizing a novel catalyst. However, this reference specifically shows that calcium and magnesium oxide showed no catalytic effect. U.S. Patents 4,210,764 and 4,223,164 likewise showed the alkoxylating effect of strontium and barium hydroxides when promoted by phenolic materials. However, these references also specifically disclose that calcium and magnesium showed no catalytic activity in these uses.

More recently European Patent Application 0026546, 0026547, and 0026544 all disclose various processes for preparing basic salts of barium and other alkaline earth metals including strontium and calcium. These references in general teach that various soluble basic salts of alkaline earth metals can be prepared which are effective catalysts for the condensation reaction products of epoxides and organic compounds having active hydrogen compounds. These references teach variously the process of carrying out such reaction as well as process of preparing these catalysts. However, these references do not show the use of magnesium as an effective catalyst and do not teach the use of the carboxylates of the present invention in the application described.

It would therefore be of great benefit to provide a process for the preparation of alkoxylates of alkyl phenols and alcohols which provide a less expensive catalyst which is innocuous in the final products and suitable for human use without catalyst removal.

It is therefore an object of the present invention to provide novel calcium and magnesium carboxylic catalysts

useful for the alkoxylation of alkyl phenols and alcohols. Other objects will become apparent to those skilled in this art as the description proceeds.

It has now been discovered according to the instant invention that alkoxylation of all classes of alcohols as well as alkyl phenols can be alkoxylated in the presence of calcium carboxylate and magnesium carboxylate catalysts or mixtures of these to provide a narrow distribution of high mole adducts while yielding a very low level of unreacted materials and undesirable by-products.

The instant invention describes a method for the alkoxylation of alcohols, alkyl phenols or mixtures of these comprising contacting said materials with an alkoxylating agent in the presence of magnesium carboxylate catalyst, calcium carboxylate catalyst or mixtures of these catalysts. The instant invention is normally carried out at temperatures of from about 90°C to about 260°C. Normally the alcohols reacted under the process of the present invention will contain from about 4 to about 24 carbon atoms, but alcohols containing from about 8 to about 16 carbon atoms are those most used for commercial purposes.

The catalysts of the present invention are magnesium or calcium carboxylates of the general formula

$$M(RCOO)_2.xH_2O$$

wherein M is calcium or magnesium and wherein R is a linear or branched acyclic group, alicyclic group, cyclic group, aryl group or hydrogen and wherein R can contain one or more carbon atoms and preferably contains from one to ten carbon atoms, and wherein x can be 0 or any number.

Representative but non-exhaustive examples of catalysts of the present invention are calcium acetate, calcium acetate dihydrate, calcium acetate monohydrate, calcium benzoate, calcium butyrate, calcium cinnamate, calcium citrate, calcium formate, calcium isobutyrate, calcium lactate, calcium laurate, calcium linoleate, calcium $\alpha$-methyl butyrate, calcium oleate, calcium palmitate, calcium

propionate, calcium stearate, calcium valerate, calcium isovalerate, calcium hexanoate, calcium octanoate, magnesium acetate, magnesium acetate dihydrate, magnesium acetate monohydrate, magnesium benzoate, magnesium butyrate, magnesium cinnamate, magnesium citrate, magnesium formate, magnesium isobutyrate, magnesium lactate, magnesium laurate, magnesium linoleate, magnesium α-methyl butyrate, magnesium oleate, magnesium palmitate, magnesium propionate, magnesium stearate, magnesium valerate, magnesium isovalerate, magnesium hexanoate, magnesium octanoate and the various hydrates of these salts.

The term "alkyl phenols" as used in the specification and claims refers to phenol itself and substituted phenols having a total of from 6 to about 36 carbon atoms, and mixtures of these materials. These materials are represented by the general formula

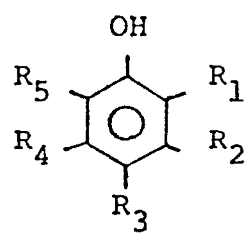

wherein $R_1$, $R_2$, $R_3$, $R_4$, and $R_5$ are, independently, hydrogen, linear or branched acyclic groups, alicyclic groups, cyclic groups, aryl groups, substituted aryl groups, halogen, hydroxyl, nitro, ether or carbonyl and wherein in addition the R-designated groups can contain one or more functionalities selected from the group consisting of halogen, nitro, carboxyl, carbonyl, amine, amide or hydroxyl.

Representative but non-exhaustive examples of phenolic-compounds which, alone or in mixture, form the alkyl phenols effectively alkoxylated in the process of the present invention are:

phenol
ortho-cresol
meta-cresol
para-cresol
2,4-dimethylphenol
2,5-dimethylphenol
2,6-dimethylphenol
2,3-dimethylphenol
ortho-chlorophenol

meta-chlorophenol
para-chlorophenol
para-nitrophenol
para-methoxyphenol
salicylic acid
meta-hydroxyacetophenone
para-aminophenol
ortho-phenylphenol
nonylphenol
octylphenol
t-butyl-para-cresol
hydroquinone
catechol
resorcinol
pyrogallol
1-naphthol
2-naphthol
4,4'-isopropylidenediphenol (bisphenol A)
methyl salicylate
benzyl salicylate
4-chloro-2-nitrophenol
para-t butylphenol
2,4-di-t-amylphenol
2,4-dinitrophenol
para-hydroxybenzoic acid
8-hydroxyquinoline
methyl para-hydroxybenzoate
2-nitro-para-cresol
ortho-nitrophenol
para-phenylphenol
phenyl salicylate
salicylaldehyde
p-hydroxy benzaldehyde
2-amino-4-chlorophenol
ortho-aminophenol
dodecylphenol
octyl cresol
dioctyl cresol
dinonylphenol
salicylamide
2,4-dichlorophenol
2,5-dichlorophenol
2,5-dichlorohydroquinone

Thus, the instant invention provides for the production of highly efficient alkyl phenol alkoxylates from phenolic materials either used alone or in mixtures in a novel, highly unexpected manner. These alkyl phenols contain from about 6 to about 36 carbon atoms although from about 6 to about 20 carbon atoms will be more common.

While the instant invention is effective with all classes of alcohols, alkanols are the preferred materials of use. Primary, secondary, tertiary, linear and branched alkanols can be used. Linear and branched primary alkanols are the most commonly used alcohols and are the preferred alcohols of the instant invention. Representative examples of such alcohols are those derived by hydrogenation of natural fats and oils, such as CO and TA alcohols, trademark of and sold by Proctor and Gamble Co., such as CO-1214N alcohol, CO 1618 alcohol, and TA 1618 alcohol, and ADOL alcohols, trademark of and sold by Ashland Oil Co., such as ADOL 54 alcohol, ADOL 61 alcohol, ADOL 64 alcohol, ADOL 60 alcohol and ADOL 66 alcohol. Alcohols produced by Ziegler chemistry can also be ethoxylated. Examples of these alcohols are ALFOL alcohols, trademark of and sold by Conoco Inc, such as ALFOL 1012 alcohol, ALFOL 1214 alcohol, ALFOL 1412 alcohol, ALFOL 1618 alcohol, ALFOL 1620 alcohol; and EPAL alcohols, trademark of and sold by Ethyl Chemical Co., such as EPAL 1012 alcohol, EPAL 1214 alcohol, EPAL 1418 alcohol. The invention is extremely useful for oxo alcohols (hydroformylation) produced from olefins. Examples of such alcohols are NEODOL alcohol, trademark of and sold by Shell Oil Co., such as NEODOL 23 alcohol, NEODOL 25 alcohol, NEODOL 45 alcohol; TERGITOL-L, trademark of Union Carbide Corp., such as TERGITOL-L 125 alcohol; LIAL alcohols, trademark of and sold by Liquichimica Co. such as LIAL 125; and isodecyl and tridecyl alcohols, sold by Exxon Corp., such as isodecyl alcohol and tridecyl alcohol. Guerbet alcohols can also be ethoxylated. Representative examples of these alcohols are STANDAMUL alcohols, trademark of and sold by Henkel Chemical Co., such as STANDAMUL GT-12 alcohol, STANDAMUL GT-16 alcohol, STANDAMUL GT-20 alcohol, STANDAMUL GT-1620 alcohol. Secondary alcohols can also be used, such as TERGITOL 15 alcohol, trademark of and sold by Union Carbide Corp.

Representative examples of such alcohols are 1-decanol; 1-undecanol; 1-dodecanol; 1-tridecanol; 1-tetradecanol 1-pentadecanol; 1-hexadecanol; 1-heptadecanol; 1-octadecanol; 1-nonadecanol; 1-eicosanol; 1-docosanol; 2-methyl-1-undecanol; 2-propyl-1-nonanol; 2-butyl-1-octanol; 2-methyl-1-tridecanol; 2-ethyl-1-dodecanol; 2-propyl-1-undecanol; 2-butyl-1-decanol; 2-pentyl-1-nonanol; 2-hexyl-1-octanol; 2-methyl-1-pentadecanol; 2-ethyl-1-tetradecanol; 2-propyl-1-tridecanol; 2-butyl-1-dodecanol; 2-pentyl-1-undecanol; 2-hexyl-1-decanol; 2-heptyl-1-decanol; 2-hexyl-1-nonanol; 2-octyl-1-octanol; 2-methyl-1-heptadecanol; 2-ethyl-1-hexadecanol; 2-propyl-1-pentadecanol; 2-butyl-1-tetradecanol; 2-pentyl-1-tridecanol; 2-hexyl-1-dodecanol; 2-octyl-1-decanol; 2-nonyl-1-nonanol; 2-dodecanol; 3-dodecanol; 4-dodecanol; 5-dodecanol; 6-dodecanol; 2-tetradecanol; 3-tetradecanol; 4-tetradecanol; 5-tetradecanol; 6-tetradecanol; 7-tetradecanol; 2-hexadecanol; 3-hexadecanol; 4-hexadecanol; 5-hexadecanol; 6-hexadecanol; 7-hexadecanol; 8-hexadecanol; 2-octadecanol; 3-octadecanol; 4-octadecanol; 5-octadecanol; 6-octadecanol; 7-octadecanol; 8-octadecanol; 9-octadecanol; 9-octadecenol-1; 2,4,6-trimethyl-1-heptanol; 2,4,6,8-tetramethyl-1-nonanol; 3,5,5-trimethyl-1-hexanol; 3,5,5,7,7-pentamethyl-1-octanol; 3-butyl-1-nonanol; 3-butyl-1-undecanol; 3-hexyl-1-undecanol; 3-hexyl-1-tridecanol; 3-octyl-1-tridecanol; 4-methyl-3-tridecanol; 2-methyl-3-undecanol; 2-methyl-4-undecanol; 4-methyl-2-undecanol; 5-methyl-2-undecanol; 4-ethyl-2-decanol; 4-ethyl-3-decanol; tetracosanol; hexacosanol; octacosanol; triacontanol; dotriacontanol; hexatriacontanol; 2-decyltetradecanol; 2-dodecylhexadecanol; 2-tetradecyloctadecanol; 2-hexadecyleicosanol

Acetylenic alcohols can also be ethoxylated using the present invention. Representative but non-exhaustive examples of such alcohols include hexynols such as

1-hexyn-3-ol, ethyloctynols such as 4-ethyl-1-octyn-3-ol, methylbutynols such as 2-methyl-3-butyn-2-ol and methylpentynols such as 3-methyl-1-pentyn-3-ol, and other alcohols such as 2,5-dimethyl-3-hexyne-2,5-diol, 3,6-dimethyl-4-octyne-3,6-diol and 2,4,7,9-tetramethyl-5 decyne-4,7-diol.

Reactions of the present invention can be carried out in the presence of any alkoxylating agent which produces a mole adduct of the sort desired. Normally such agents are alpha or beta alkylene oxides. In most commercial operations either ethylene oxide, propylene oxide or mixtures of these will be used to produce an adduct. Of these materials ethylene oxide is most preferred.

The reaction products produced by the process of the present invention can have any desired content of alkoxylating agent. Utilizing ethylene oxide as an example such reaction products will normally range from about 20 to about 80 percent content of ethylene content (EO) based on weight. The amount of EO present in the reaction is not critical other than a minimum amount necessary to provide sufficient units to meet the mole adduct level desired for the material being reacted.

The instant invention is normally carried out at temperatures of from about 90 to 260°C. Temperatures of from about 120°C to about 260°C are preferred. For most practical purposes commercial operations will normally be carried out in the temperature range of from about 150°C to about 200°C. The most preferred temperature range is from 160°C to about 190°C.

The process of the instant invention can be carried out at ambient pressure. Pressures below about 60 pounds per square inch gauge (psig) are preferred. However, pressures above and below this range can be used. Pressure or lack of pressure is not a detriment to the process of the instant invention and can be adjusted to suit the individual process. Normally it is simply more convenient to carry out the reaction in the pressure range of from about atmospheric to about 100 psig.

When used, the catalysts or catalyst mixtures of the present invention can be used in any desired quantity. The larger the quantity used the more quickly the reaction goes to completion, although larger quantities do not appear to significantly alter the distribution obtained. However, for practical purposes, normally at least about 0.05 weight percent catalysts based upon the weight of the material to be reacted would be present in the reaction. But normally from about 0.1 to about 5.0 weight percent is preferred, and from about 0.1 to about 2.0 weight percent is most preferred. The amount of catalyst or catalyst mixture which should be present is in general an effective amount and can be adjusted according to the dictates of a particular reaction carried out.

The process of the present invention can be carried out by simply adding the catalyst to the combination of the alkyl phenols or alcohols and alkoxylating agent under sufficient conditions for alkoxylation to occur. However, in order to further increase catalytic activity, these catalysts may be activated by simply heating the catalyst together with a small quantity of the material to be adducted for a time ranging up to about 2 hours and at a temperature ranging up to about 175°C.

For handling ease, a solvent effective to dissolve the catalyst can be used. Such solvents are entirely optional and not critical in any manner to the present invention.

The invention is more concretely described with reference to the examples below wherein all parts and percentages are by weight unless otherwise specified. The examples are provided to illustrate the instant invention and not to limit it.

Example 1

A stainless steel reactor was charged with 120 grams of ALFOL 1214 alcohol (a 12-14 carbon atom alcohol, trademark of and produced by Conoco Inc.), 1.0 grams of $Ca(OH)_2$ and 1 gram of 2-ethylhexanoic acid. After purging with nitrogen at 500 cubic centimeters (cc) per minute for 30 minutes at a temperature of 150°C, the reactor was evacuated and the temperature range raised to 175°C. Ethylene oxide (EO) was then introduced to a total pressure of about 40 psig and EO uptake of 150 grams was allowed to proceed at this pressure requiring a reaction time of 160 minutes. After reaction the catalyst was neutralized. The product had a 50°F pourpoint and 2.67 weight percent free alcohol.

Example 2

The experiment was carried out exactly as described in Example 1, except that 0.1 grams of potassium hydroxide was used. The reaction time was 83 minutes, yielding a product with a 60°F pour point and 4.33 weight percent free alcohol.

Example 3

The experiment was carried out exactly as described in Example 1 except that 0.82 grams calcium acetate was used as a catalyst. The reaction time was 137 minutes, yielding a product having a free alcohol content of 3.06%.

Example 4

As a comparison, an attempt was made to ethoxylate the materials of example 1 in the same fashion as described in example 1 except that calcium metal was used as the catalyst. No appreciable ethylene oxide uptake occurred in 3 hours.

Example 5

As a comparison, an attempt was made to ethoxylate the materials of example 1 in the same fashion as described in example 1 except that 2 grams calcium hydroxide was used as the catalyst. No appreciable ethylene oxide uptake occurred in 3 hours.

Example 6

As a comparison, an attempt was made to ethoxylate the materials of example 1 in the same fashion as described in example 1 except that 2 grams $Ca(OH)_2$ and 2.5 grams phenol (calcium hydroxide promoted with phenol) was used as the catalyst. No appreciable ethylene oxide uptake occurred in 3 hours.

Example 7

As a comparison, an attempt was made to ethoxylate the materials of example 1 in the same fashion as described in example except that calcium hydroxide in the presence of adipic and succinic acid was used as the catalyst. No appreciable ethylene oxide uptake occurred in 3 hours.

Example 8

An experiment was carried out exactly as described in example 1 except that 3.0 grams $Mg(OAc)_2 \cdot 4H_2O$ was used as the catalyst. Ethylene oxide uptake was allowed to proceed to 180 grams total requiring 315 minutes. After neutralization the product was recovered. The ethoxylate had a pour point of 65°F and a free alcohol level of 1.2 weight percent.

Example 9

An experiment was repeated exactly as described in example 8 except that 0.1 grams of sodium hydroxide was used. The ethoxylate recovered had a pour point of 75°F and a free alcohol content of 3.6 weight percent.

Example 10

The reaction described in example 8 was repeated exactly using 1 gram magnesium metal as a catalyst. No significant ethoxylation was found.

Example 11

The reaction described in example 8 was repeated exactly using 1 gram magnesium hydroxide as a catalyst. No significant ethoxylation was found.

BAD ORIGINAL

Example 12

The reaction described in example 8 was repeated exactly using 1 gram magnesium hydroxide together with 7.7 grams phenol promoter as a catalyst. No significant ethoxylation was found.

Example 13

Magnesium 2-ethyl hexanoate was prepared by refluxing a mixture of 5 grams magnesium hydroxide, 12.4 grams 2-ethyl hexanoic acid and 30 grams Formula 30 alcohol (Denatured alcohol described in monograph no. 212, pg 31, THE MERCK INDEX, 9th edition, Merck & Co. Inc., 1976) overnight Excess solvent was then removed by thin film evaporation to yield 15.3 grams catalyst. Thereafter 150 grams of NEODOL 25 alcohol together with 3.1 grams of the catalyst prepared were used exactly in the fashion described in example 8 to produce a 50% ethoxylate (150 grams EO in 410 minutes). The product had a pour point of 50°F and a free alcohol level of 4.3 weight percent.

Example 14

As a comparison, 150 grams of NEODOL 25 alcohol was reacted with 150 grams of ethylene oxide in the presence of 0.25 grams potassium hydroxide catalyst in exactly the same fashion as described in example 8. The resulting ethoxylate had a pour point of 60°F and a free alcohol level of 6.8%.

Example 15

An alkoxylation catalyst was preformed by the addition of 50 grams Formula 30 alcohol to a 500 ml 1 neck flask followed by the addition of 9.7 grams of 2-ethylhexanoic acid and then 5.0 grams of $Ca(OH)_2$. Additional Formula 30 alcohol was added to facilitate stirring and the mixture was refluxed and stirred overnight. The flask containing the mixture was then placed on a Rotavapor-R thin film evaporator and taken to dryness. The preformed catalyst product weighed 13.5 grams.

Example 16

An experiment was carried out as described in Example 15 except 5 grams $Ca(OH)_2$ and 8.1 grams acetic acid were used. Enough Formula 30 alcohol was used to facilitate mixing. The recovered product weighed 11.5 grams.

Example 17

An experiment was carried out as described in Example 15 except 5 grams $Mg(OH)_2$ and 12.4 grams 2-ethylhexanoic acid were used. The product weighed 15.3 grams.

Example 18

An experiment was carried out as described in Example 15 except 5 grams $Mg(OH)_2$ and 5.1 grams acetic acid were used. The recovered product weighed 8.8 grams.

Example 19

An experiment was carried out as described in Example 15 except 5 grams $Ca(OH)_2$, 4 grams acetic acid and 9.7 grams 2-ethylhexanoic acid were used. 16.7 grams of product were recovered.

Example 20

An experiment was carried out as described in Example 15 except 5 grams $Ca(OH)_2$ and 20.2 grams HYDREX 450 (stearic acid) were used. The recovered product weighed 24.0 grams.

Example 21

A 600 cubic centimeter (cc) stainless steel reactor is charged with 150 grams of nonylphenol and 4 grams of $Mg(OAc)_2.4H_2O$ catalyst. After purging with nitrogen at 250 cubic centimeters (cc) per minute for 1 hour at a temperature of 150°C, the reactor is evacuated and the temperature raised to about 170°C. Ethylene oxide (EO) is then introduced to a total pressure of about 40 pounds per square inch gauge (psig) and EO uptake of 150 grams is allowed to proceed at this pressure. After ethoxylation, the catalyst was neutralized. Ethoxylation requires about 350 minutes for completion.

BAD ORIGINAL

Example 22

An experiment is carried out as described in Example 21 except that 150 grams of 2,3-dimethylphenol and 1.6 grams of preformed catalyst as described in Example 15 are used. Ethoxylation requires about 200 minutes for completion.

Example 23

An experiment is carried out as described in Example 22 except 150 grams of propylene oxide (PO) is used.

While certain embodiments and details have been shown for the purpose of illustrating this invention, it will be apparent to those skilled in this art that various changes and modifications may be made herein without departing from the spirit or scope of the invention.

We claim:

1. A method for the alkoxylation of alcohols and alkyl phenols of the general formula

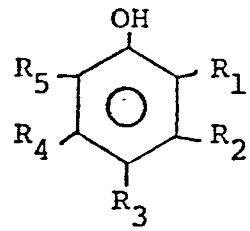

wherein $R_1$, R, $R_3$, $R_4$ an $R_5$ are, independently, hydrogen, halogen, or alkyl groups containing from 1 to 20 carbon atoms in the presence of calcium carboxylate and magnesium carboxylate catalysts or mixtures of these to provide a narrow distribution of high mole adducts while yielding a very low level of unreacted materials and undesirable by-products, or mixtures of these comprising contacting said materials with an alkoxylating agent in the presence of a catalyst of the general formula $M(RCOO)_2.XH_2O$ where M is magnesium or calcium and wherein R is a linear or branched acyclic group, alicyclic group, cyclic group, aryl group or hydrogen and wherein R can contain one or more carbon atoms and preferably contains from one to ten carbon atoms, and wherein X can be 0 or any number at a temperature of from about 90°C to about 260°C.

2. A method as described in claim 1 wherein the alkoxylation is carried out using ethylene oxide, propylene oxide or mixtures of these.

3. A method as described in claim 2 wherein the catalyst is selected from the group consisting of calcium acetate, magnesium acetate, calcium octanoate, magnesium octanoate, calcium hexanoate, magnesium hexanoate, and hydrates of these salts.

4. A method as described in claim 3 wherein the reaction is carried out at a pressure of up to about 100 pounds per square inch gauge.

5. A method as described in claim 4 wherein ethylene oxide, propylene oxide or mixtures thereof are used as the alkoxylating agent and a mole adduct ratio ranging from about 20 weight percent to about 80 weight percent of the alkoxylated product is reached.

6. A method as described in claim 5 wherein the magnesium and calcium carboxylate catalysts are present in an amount ranging from about 0.05 to about 5.0 weight percent based upon the alkyl phenol or alcohol to be reacted.

7. A method as described in claim 2 when carried out in a continuous reaction.

0085167

European Patent Office

# EUROPEAN SEARCH REPORT

Application number

EP 82 11 1641

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| D,A | EP-A-0 026 546 (UNION CARBIDE) * Claims 1, 7 * --- | 1,3 | C 07 C 41/03<br>B 01 J 31/04 |
| A | FR-A-2 322 840 (BEROL KEMI) * Claims 1, 3 * --- | 1,2 | |
| A | Patent Abstracts of Japan vol. 1, no. 102, 10 September 1977, page 2240C77 & JP-A-52-62 232 ----- | 1,3 | |

TECHNICAL FIELDS SEARCHED (Int. Cl. 3)

B 01 J 31/04
C 07 C 41/01
C 07 C 41/03

The present search report has been drawn up for all claims

| Place of search | Date of completion of the search | Examiner |
|---|---|---|
| BERLIN | 17-03-1983 | KNAACK M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document